# EUROPÄISCHE PATENTANMELDUNG

(11) **EP 2 354 396 A2**
(43) Veröffentlichungstag der Anmeldung: **10.08.2011**
(21) Anmeldenummer: 11153359.2
(22) Anmeldetag: 04.02.2011
(51) Int. Cl.: E05C 19/14, A61L 2/26, E05B 47/02

(54) **Vorrichtung zur Verriegelung einer Tür einer Reinigungsvorrichtung, Desinfektionsvorrichtung, Sterilisationsvorrichtung oder Reinigungs- und Desinfektionsvorrichtung**

(30) Priorität: 05.02.2010 DE 202010002220 U; 12.02.2010 DE 202010002413 U
(71) Anmelder: Melag oHG, 10829 Berlin (DE)
(72) Erfinder: Hörning, Andreas Robert, Dipl.-Ing. (FH), 10969 Berlin (DE); Litzba, Guido, Dipl.-Ing. (FH), 12161 Berlin (DE)
(74) Vertreter: Sokolowski, Fabian

(57) **Zusammenfassung**

Die vorliegende Erfindung betrifft eine Verriegelungsvorrichtung für eine Tür einer Reinigungsvorrichtung, Desinfektionsvorrichtung, Sterilisationsvorrichtung oder Reinigungs- und Desinfektionsvorrichtung. Die Erfindung zeichnet sich durch mindestens eine Sperrklinke (2), mindestens eine Schubstange (3) und mindestens einen Exzenter (4) aus, die durch Drehgelenke (A, B) miteinander verbunden sind, wobei bei geschlossener Tür die Drehgelenke (A, B) so angeordnet sind, dass bei einer von außen auf die Tür aufgebrachten Zugkraft ein Drehmoment auf den Exzenter (4) aufgebracht wird, das in der Verschlussrichtung (V) wirkt, so dass ein gesicherter Verschlusszustand vorliegt.

## Beschreibung

Die vorliegende Erfindung betrifft eine Vorrichtung zur Verriegelung einer Tür einer Reinigungsvorrichtung, Desinfektionsvorrichtung, Sterilisationsvorrichtung oder Reinigungs- und Desinfektionsvorrichtung gemäß dem Oberbegriff des Anspruchs 1.

Der Bedarf an Reinigungsvorrichtungen, insbesondere an Reinigungs- und Desinfektionsvorrichtungen auf dem gewerblichen Gebiet ist steigend. Gerade in Arztpraxen oder in kleinen chemischen und analytischen Laboren werden verstärkt Reinigungs- und Desinfektionsvorrichtungen eingesetzt, die ständig steigende Anforderungen erfüllen müssen.

Gerade bei Dampfsterilisatoren ist ein sicherer druck- und gasdichter Verschluss des Sterilisators wichtig, um den Sterilisationsdruck in der Sterilisationskammer aufrechtzuerhalten und ein Entweichen von Dämpfen zu verhindern.

Der Erfindung liegt das Problem zu Grunde, eine sichere und kosten- und platzsparende Verriegelungsvorrichtung zu stellen.

Diese Aufgabe wird durch die Merkmale des Anspruchs 1 gelöst.

Durch die Verwendung mindestens einer Sperrklinke, mindestens einer Schubstange und mindestens eines Exzenters, die durch Drehgelenke miteinander verbunden sind, wird erreicht, dass bei geschlossener Tür die Drehgelenke so angeordnet sind, dass bei einer von außen auf die Tür aufgebrachten Zugkraft ein Drehmoment auf den Exzenter aufgebracht wird, dass in der Verschlussrichtung wirkt, so dass ein gesicherter Verschlusszustand vorliegt. Der gesicherte Verschlusszustand ist ohne Weiteres nicht aufhebbar, insbesondere nicht durch starkes Ziehen an der Tür.

Dabei ist es vorteilhaft, wenn die Sperrklinke mittels einer Drehbewegung in Verschlussrichtung mit einem entsprechenden Verschlussgegenstück der Tür verbindbar ist. Damit kann z.B. ein sicheres Einrasten gewährleistet werden.

Auf den Exzenter ist vorteilhafterweise zum Öffnen oder Schließen der Tür ein Drehmoment mittels eines Übertragungsmittels, mit Vorteil mittels einer Welle, übertragbar.

Dabei ist es vorteilhaft, wenn das Drehmoment über ein Betriebsmittel, insbesondere einen Elektromotor, auf das Übertragungsmittel, insbesondere auf die Welle, übertragbar ist. Mit Vorteil weist das Betriebsmittel keine Selbsthemmung auf.

Vorteilhafterweise kann das Übertragungsmittel und/oder das Betriebsmittel eine Lagerung aufweisen, die federnd und/oder dämpfend wirkt.

Das Drehmoment des Exzenters zum Öffnen oder Schließen der Tür wird vorzugsweise durch die Schubstange auf die Sperrklinke übertragen. Die Übertragung erfolgt mit Vorteil im gleichen Drehsinn.

Die Übertragung des Drehmoments auf die Sperrklinke ist nur bis zu einem bestimmten Drehwinkel, vorteilhafterweise 90°, möglich. Innerhalb dieses Drehwinkels, befindet sich die Verriegelungsvorrichtung in einem ungesicherten Verschlusszustand. Durch eine Umkehrung des Drehmoments auf den Exzenter kann die Verriegelungsvorrichtung wieder in den geöffneten Zustand überführt werden. Weiterhin ist es in dem ungesicherten Verschlusszustand grundsätzlich möglich, durch eine Krafteinwirkung auf die Sperrklinke, beispielsweise durch Ziehen an der Tür, ein Drehmoment entgegen der Verschlussrichtung auf den Exzenter zu übertragen und die Verriegelungsvorrichtung zu öffnen. Der ungesicherte Verschlusszustand ist bei einem betriebsgemäßen Öffnen und Schließen der Verriegelungsvorrichtung durch die Drehmomentübertragung auf den Exzenter nur ein temporärer Zustand.

Die Sperrklinke ist nach Erreichen des ungesicherten Verschlusszustandes in der Verschlussrichtung nur noch bedingt drehbar. Der Exzenter und die Schubstange werden durch eine weitere Wirkung des Drehmoments in Verschlussrichtung gedreht. Dadurch wird ein gesicherter Verschlusszustand erreicht. Die Sperrklinke wird dadurch anordnungsbedingt kurzfristig etwas über den erreichten Drehwinkel hinaus gedreht, um danach wieder die Ausgangslage zu erreichen. Die erreichten Drehwinkel der Sperrklinke entsprechen beispielsweise 90°-91 °-90 °.

In der nun erreichten Anordnung des Exzenters und der Schubstange ist es nicht mehr möglich über die Sperrklinke ein Drehmoment entgegen der Verschlussrichtung zu übertragen. Jede Krafteinwirkung auf die Sperrklinke führt nun nicht mehr zu einer Kraftübertragung entgegen sondern nur noch in der Verschlussrichtung.

Durch diese vorteilhafte Anordnung ist es nicht mehr möglich, mittels einer Kraftübertragung auf die Sperrklinke, wie beispielsweise durch Ziehen an der Tür, die Verriegelungsvorrichtung zu öffnen. Dies ist nur noch durch eine Umkehrung des Drehmoments am Exzenter möglich.

Mit Vorteil sind am Exzenter Sicherungskanten ausgebildet. Sie wirken beim Erreichen des geöffneten (ungesicherten) und des gesicherten Verschlusszustands mit den entsprechenden Anschlägen am Gehäuse zusammen und kompensieren eine weitere Krafteinwirkung durch den Exzenter.

Mit Vorteil wird bei einer Verwendung eines Betriebsmittels durch das Zusammenwirken der Sicherungskanten und den Anschlägen der Widerstand beim Erreichen des geöffneten und des gesicherten Verschlusszustands und somit der Stromfluss erhöht. Mit Vorteil wird das Betriebsmittel durch Messung der Stromstärke und/oder Spannung am Betriebsmittel beim Erreichen einer Obergrenze, vorteilhafterweise automatisch, ausgeschaltet.

Es ist von Vorteil, wenn am Gehäuse eine Anschlagkante angebracht ist, welche mit einer Innenwand der Vorrichtung zusammenwirkt um die Kräfte, die beim Erreichen des gesicherten Zustandes auf die Verriegelungsvorrichtung wirken, zu kompensieren.

Vorteilhafterweise wird beim Erreichen des gesicherten Verschlusszustandes ein Kontaktschalter mittels einer Exzenterkante betätigt und ein optisches und/oder haptisches und/oder akustisches Signal ausgelöst das den gesicherte Verschlusszustand angezeigt.

In einer vorteilhaften Ausführungsform enthält die Verriegelungsvorrichtung ein Notentriegelungssystem zum Auflösen des gesicherten Verschlusszustandes, mit dem von außen eine Notentriegelungskraft aufbringbar ist, mit dem der Exzenter und/oder die Schubstange aus dem gesicherten Verschlusszustand bringbar sind. Dies ermöglicht mit Vorteil ein im Notfall schnelles Öffnen unabhängig von eventuellen Betriebsmitteln. Dadurch ist es beispielsweise bei Stromausfall möglich die entsprechende Vorrichtung dennoch öffnen zu können.

Der gesicherte Verschlusszustand wird in den ungesicherten durch Krafteinwirkung von außen überführt. Dies wird mit Vorteil mittels eines Notentriegelungsauslösers erreicht. Auf die Schubstange und/oder den Exzenter wird eine Notentriegelungskraft vorteilhafterweise mittels Hebelkraft über einen Notentriegelungshebel übertragen. Weitere Kraftübertragungen wie beispielsweise mittels Zugkraft über eine Zugvorrichtung sind ebenso denkbar.

In einer alternativen Ausgestaltung weist der Verriegelungsmechanismus ein Notentriegelungssystem auf, mit dem von außen ein Drehmoment aufbringbar ist, mit dem die Sperrklinke in den ungesicherten Verschlusszustand oder einen geöffneten Zustand bringbar ist.

In einer vorteilhaften Ausführungsform erfolgt die Krafteinwirkung von außen über mindestens einen Notentriegelungsauslöser, insbesondere mindesten einen Zuganker. Die Kraftübertragung wird vorteilhafterweise mittels eines Notentriegelungshebels innerhalb einer Zwangsführung, vorteilhafterweise in einem Zwangsführungskäfig und/oder einer Zwangsführungskante an der Schubstange, erreicht. Mit anderen Worten ist vorzugsweise mittels mindestens eines Notentriegelungshebels, der innerhalb einer Zwangsführung gelagert ist, insbesondere in einem Zwangsführungskäfig und/oder an einer Zwangsführungskante der Schubstange, die Krafteinwirkung von außen auf die Schubstange und/oder auf den Exzenter übertragbar.

In einer weiteren vorteilhaften Ausführungsform wird der Ausgangszustand des Notentriegelungssystems durch ein Rückführungsmechanismus am Notentriegelungshebel beispielsweise über eine Feder wieder hergestellt.

Durch eine Krafteinwirkung auf den Notentriegelungsauslöser wird die Verriegelungsvorrichtung wieder in den ungesicherten Verschlusszustand überführt. Es ist nun möglich durch eine Krafteinwirkung auf die Sperrklinke, beispielsweise durch Ziehen an der Tür, ein Drehmoment entgegen der Verschlussrichtung auf den Exzenter zu übertragen und die Verriegelungsvorrichtung zu öffnen.

Die Kraftübertragung mittels des Notentriegelungsauslösers kann sowohl von Hand, aber auch mittels eines Notentriegelungsbetriebsmittels, vorteilhafterweise einem Elektromotor, erfolgen.

Durch eine formschlüssige Verbindung von Antriebswelle und Exzenter, insbesondere durch ein abgeflachtes Wellenende, einen Vierkant oder einen Sechskant lassen sich auf einfache Weise relativ hohe Drehmomente übertragen.

Die mechanische Stabilität wird erhöht, indem die Schubstange und der Exzenter im Zusammenbau mindestens teilweise Kontaktflächen zur gegenseitigen Abstützung aufweisen. Durch das Aneinanderliegen führen sich die Bauteile gegenseitig.

Weitere Merkmale und Vorteile der Erfindung werden bei der nachfolgenden Beschreibung einer Ausführungsform unter Bezugnahme auf die Figuren verdeutlicht.

Es zeigen:
Fig. 1 eine schematische Seitenansicht des Aufbaus einer Verriegelungsvorrichtung mit Notentriegelungssystem, insbesondere einer Reinigungs- und Desinfektionsvorrichtung, in geöffnetem Zustand
Fig. 2 eine schematische Seitenansicht des Aufbaus einer Verriegelungsvorrichtung mit Notentriegelungssystem, insbesondere einer Reinigungs- und Desinfektionsvorrichtung im ungesicherten Verschlusszustand
Fig. 3 eine schematische Seitenansicht des Aufbaus einer Verriegelungsvorrichtung mit Notentriegelungssystem, insbesondere einer Reinigungs- und Desinfektionsvorrichtung, im gesicherten Verschlusszustand
Fig. 4 eine schematische Innenansicht des Aufbaus einer Verriegelungsvorrichtung mit Notentriegelungssystem, insbesondere einer Reinigungs- und Desinfektionsvorrichtung, zur Verdeutlichung der Lagerung des Übertragungsmittels und des Notentriegelungssystems
Fig. 5 eine schematische Innenansicht des Aufbaus einer Verriegelungsvorrichtung mit Notentriegelungssystem, insbesondere einer Reinigungs- und Desinfektionsvorrichtung, zur Verdeutlichung der Lagerung des Übertragungsmittels und des Notentriegelungssystems
Fig. 6 eine schematische Seitenansicht des Aufbaus einer Verriegelungsvorrichtung mit Notentriegelungssystem, insbesondere eines einer Reinigungs- und Desinfektionsvorrichtung, zur Verdeutlichung der Lagerung des Übertragungsmittels und des Notentriegelungssystems;
Fig. 7 eine Detaildarstellung einer Verbindung zwischen Exzenter und Welle.

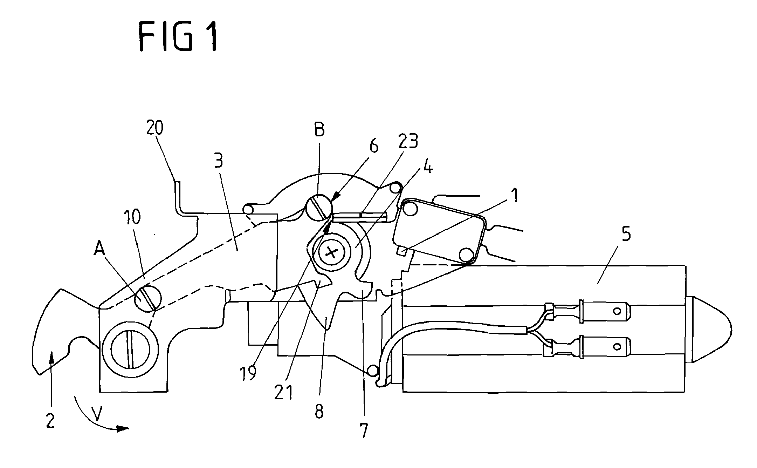

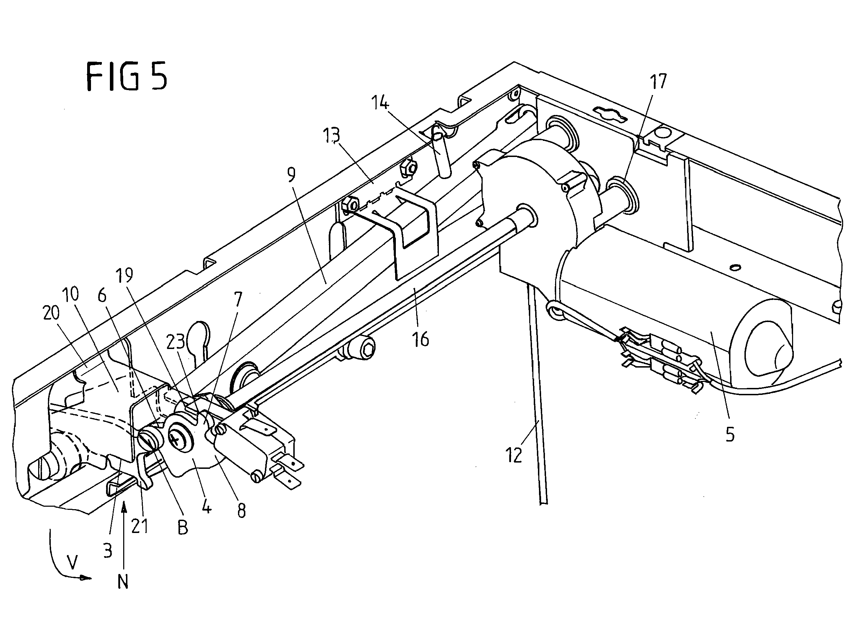

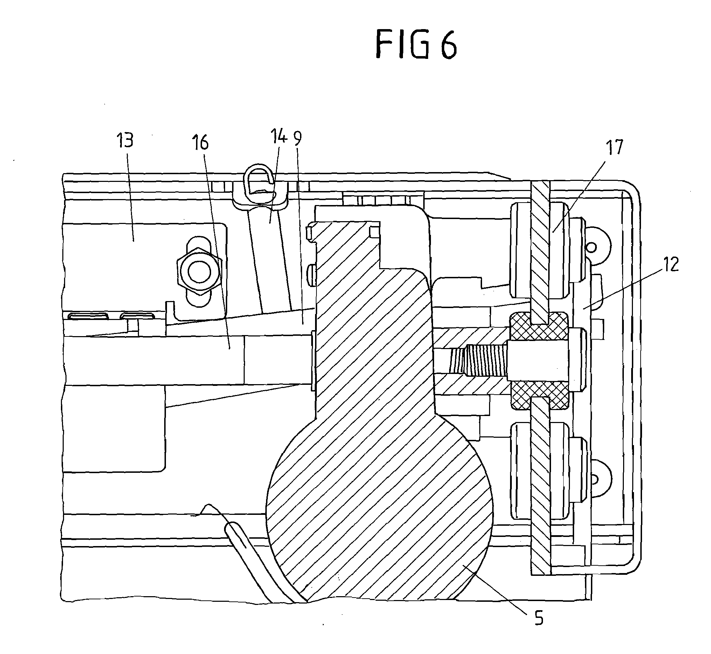

Die Fig. 1 bis 6 verdeutlichen den Aufbau und Funktionsweise eines Ausführungsbeispiels einer Verriegelungsvorrichtung mit Notentriegelungssystem. Die Fig. 1 bis 4 zeigen hauptsächlich die Funktionsweise der Verriegelungsvorrichtung, wohingegen Fig. 5 und 6 das Notentriegelungssystem und die Lagerung des Übertragungsmittels 15 veranschaulichen. Einzelheiten einer beispielsweise Reinigungs- und Desinfektionsvorrichtung werden hier aus Gründen der Übersichtlichkeit nicht dargestellt. Die Verriegelungsvorrichtung ist im vorliegenden Fall in einer Reinigungs- und Desinfektionsvorrichtung angeordnet. Diese verriegelt dann eine Tür, die eine Öffnung der Reinigungs- und Desinfektionsvorrichtung verschließt. Alternativ kann die Verrieglungsvorrichtung auch in einer Reinigungsvorrichtung, Desinfektionsvorrichtung und Sterilisationsvorrichtung angeordnet sein

In Fig. 1 ist die Verriegelungsvorrichtung in geöffneten Zustand dargestellt. Dies ist dadurch erkennbar, dass eine Sperrklinke 2 sich in der Stellung "offen" befindet. Im verschlossenen Zustand ist die Sperrklinke 2 gegenüber dem offenen Zustand entgegen dem Uhrzeigersinn verschwenkt.

Die Verriegelungsvorrichtung weist neben der Sperrklinke 2, eine Schubstange 3 und einen Exzenter 4 innerhalb eines Gehäuses 10 auf, welche durch Drehgelenke A, B miteinander verbunden sind.

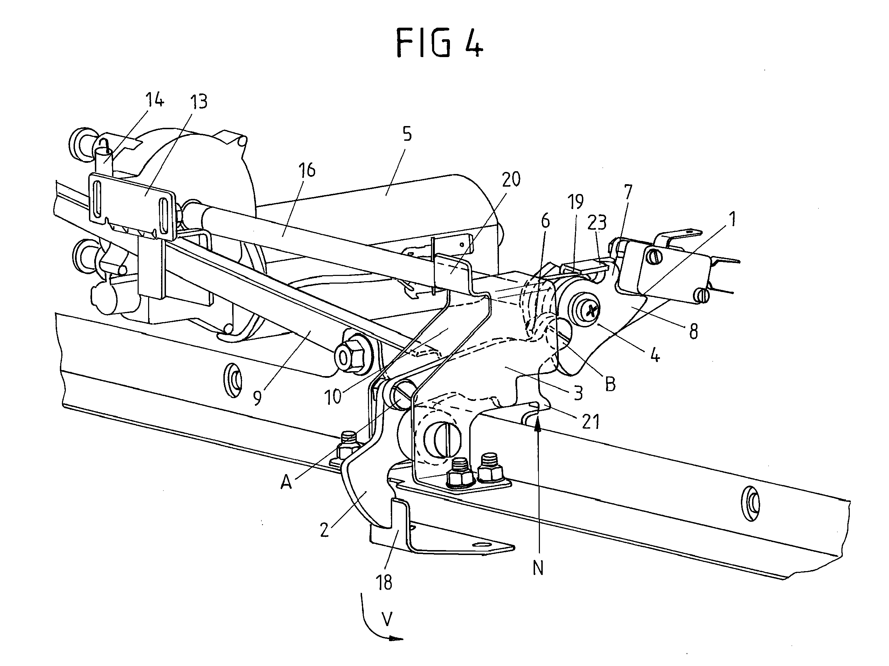

Zum Öffnen oder Schließen der Tür der Reinigungs- und Desinfektionsvorrichtung wird ein Drehmoment über ein Übertragungsmittel 15, vorteilhafterweise durch eine Welle 16, auf den Exzenter 4 übertragen. Die Welle 16 ist in dieser Figur nicht erkennbar und wird in Fig. 4 bis 6 verdeutlicht. Der Exzenter 4 überträgt das Drehmoment über die Schubstange 3 auf die Sperrklinke 2. Die Übertragung des Drehmoments erfolgt mit Vorteil im gleichen Drehsinn, ist aber grundsätzlich in oder entgegen der Verschlussrichtung V möglich.

Vorteilhafterweise wird durch ein Betriebsmittel 5, insbesondere einen Elektromotor, das Drehmoment auf das Übertragungsmittel 15, insbesondere auf die Welle 16, übertragen. Das Betriebsmittel 5 ist ebenfalls hier nicht erkennbar wird aber in Fig. 4 bis 6 verdeutlicht. Alternativ sind aber auch magnetische, hydraulische oder pneumatische Antriebe als Betriebsmittel 5 verwendbar.

Mit Vorteil weist das Übertragungsmittel 15, insbesondere die Welle 16, eine Lagerung 17 auf die eine Federung und Dämpfung ermöglicht. Die Lagerung 17 ist ebenfalls hier nicht erkennbar wird aber in Fig. 5 und 6 verdeutlicht.

Durch eine Übertragung des Drehmoments des Exzenters 4 auf die Sperrklinke 2, die in einer vorteilhaften Ausführungsform die Form eines Hakens aufweist, vollführt die Sperrklinke 2 eine Drehbewegung in Verschlussrichtung V (entgegen dem Urzeigersinn). Dadurch wird sie mit einem entsprechenden Verschlussgegenstück 18 (hier nicht erkennbar aber in Fig. 4 abgebildet) der Tür verbindbar.

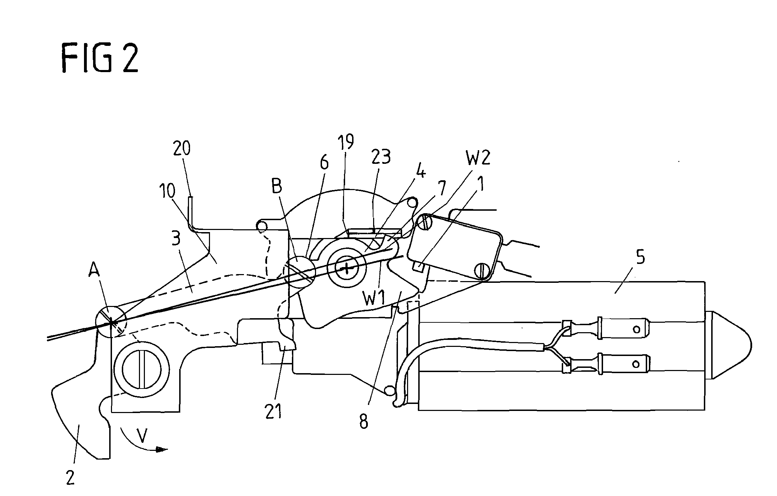

Die Funktionsweise der Verriegelungsvorrichtung wird in Fig. 2 weiter verdeutlicht. Die Fig. 2 stellt einen zunächst ungesicherten Verschlusszustand des Systems dar.

Dabei ist es sinnvoll, wenn die Übertragung des Drehmoments des Exzenters 4 über die Schubstange 3 auf die Sperrklinke 2 für die Erreichung eines zunächst nur ungesicherten Verschlusszustandes nur bis zu einem bestimmten Drehwinkel, vorteilhafterweise bis zu 90°, erfolgt. Die Sperrklinke 2 ist dann mit dem Verschlussgegenstück 18 (siehe Fig. 4) verbunden. Der ungesicherte Verschlusszustand ist beim betriebsgemäßen Öffnen und Schließen der Verriegelungsvorrichtung nur ein Übergangszustand.

Zur Verdeutlichung der Funktion dieser Ausführungsform ist in Fig. 2 eine erste Wirklinie W1 für angreifende Kräfte zwischen der Anlenkung der Sperrklinke 2 und dem Exzenter 4 dargestellt. Im ungesicherten Verschlusszustand liegt eine zweite Wirklinie W2 für Kräfte, die durch die Mitten der beiden Gelenke der Schubstange 3 geht, oberhalb der ersten Wirklinie W1.

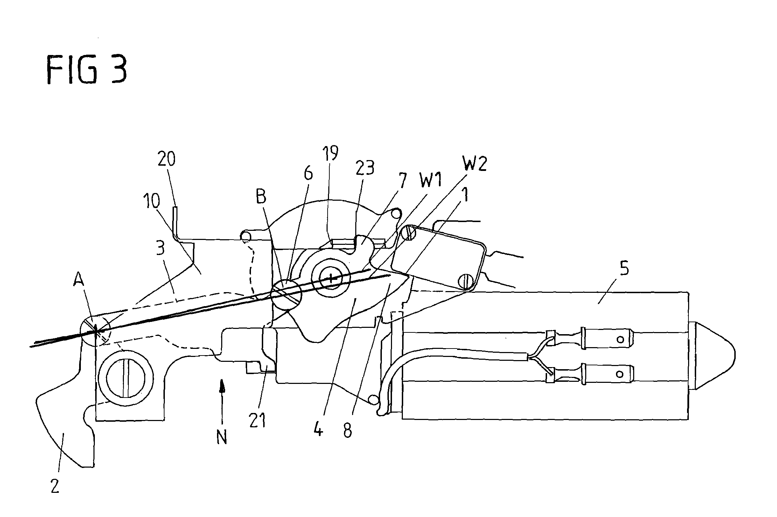

Die Funktionsweise der Verriegelungsvorrichtung wird in Fig. 3 weiter verdeutlicht. Die Fig. 3 stellt einen gesicherten Verschlusszustand des Systems dar.

In Fig. 3 sind ebenfalls die in Fig. 2 dargestellten Wirklinien W1, W2 eingetragen. Dabei wird deutlich, dass die zweite Wirklinie W2 nunmehr unterhalb der ersten Wirklinie W1 liegt. In diesem Zustand liegt der gesicherte Verschlusszustand vor.

Zum besseren Verständnis ist in Fig. 4 eine weitere Seitenansicht dieses Zustandes abgebildet. Sie verdeutlicht unter anderem das Funktionsprinzips des Übertragungsmittels 15, insbesondere der Welle 16.

Die Sperrklinke 2 ist nach Erreichen des ungesicherten Verschlusszustandes mit dem Verschlussgegenstück 18 der Tür verbunden (siehe Fig. 2). Durch ein weiteres Drehmoment auf den Exzenter 4 wird eine weitere Drehung des Exzenters 4 und der Schubstange 3 in Verschlussrichtung V erreicht. Dadurch wird das System in einen gesicherten Verschlusszustand überführt. Die Sperrklinke 2, da sie mit der Schubstange 3 und dem Exzenter 4 verbunden ist, wird anordnungsbedingt kurzfristig etwas über den erreichten Drehwinkel hinaus gedreht, um danach wieder die Ausgangslage zu erreichen. Die erreichten Drehwinkel der Sperrklinke 2 entsprechen beispielsweise 90°-91°-90°.

Durch diese in Fig. 3 verdeutlichte Anordnung des Exzenters 4 und der Schubstange 3 ist es nicht mehr möglich, über die Sperrklinke 2 ein Drehmoment auf den Exzenter 4 entgegen der Verschlussrichtung V zu übertragen (Kniehebel-Prinzip). Jede Krafteinwirkung (z.B. ein Ziehen an der Tür) auf die Sperrklinke 2 führt in dem gesicherten Verschlusszustand nicht zu einer Übertragung eines Drehmoments auf den Exzenter 4 entgegen der Verschlussrichtung V, sondern zu einer Übertragung in Verschlussrichtung V.

Mit Vorteil weist das Betriebsmittel 5, beispielsweise einen 2 Nm starken Elektromotor, zur Übertragung des Drehmoments auf das Übertragungsmittel 15, insbesondere auf die Welle 16, ein großes Drehmoment auf. Da die Hebelkräfte an der Schubstange 3 und dem Exzenter 4 kurz vor dem Erreichen des gesicherten Verschlusszustands am schlechtesten sind wird durch ein großes Drehmoment an der Welle 16 dem entgegengewirkt. Dadurch wird ein sicheres, schnelles und komfortables Schließen der Tür gewährleistet.

Durch diese vorteilhafte Anordnung des Exzenters 4 und der Schubstange 3 ist es nicht mehr möglich durch eine Krafteinwirkung auf die Sperrklinke 2, wie beispielsweise durch Ziehen an der Tür, das System zu öffnen. Das System kann nun nur noch durch eine Umkehrung des Drehmoments am Exzenter 4, entgegen der Verschlussrichtung V, in einen geöffneten Zustand überführt werden.

In einer vorteilhaften Ausführungsform besitzt der Exzenter 4 eine entsprechend geformte Sicherungskante 6 für den geöffneten Verschlusszustand und eine entsprechend geformte Sicherungskante 7 für den gesicherten Verschlusszustand mit den entsprechenden Anschlägen 19 und 23 am Gehäuse 10, um eine weitere Krafteinwirkung über den Exzenter 4 in den entsprechenden Verschlusszuständen zu kompensieren. Dadurch wird einer Beschädigung, beispielsweise der Halterung des Kontaktschalters 1, entgegengewirkt.

Mit Vorteil wird durch ein Zusammenwirken der Sicherungskanten 6 und 7 mit den Anschlägen 19 und 23 in den entsprechenden Verschlusszuständen der Widerstand und damit die Stromstärke und Spannung in dem Betriebsmittel 5 erhöht. Durch Messung der Stromstärke und/oder der Spannung am Betriebsmittel 5 ist es vorteilhafterweise möglich beim Erreichen einer Obergrenze des Stromflusses das Betriebsmittel 5, mit Vorteil automatisch, auszuschalten. Ein Betriebsmittel 5, das beispielsweise einen starken Elektromotor aufweist, ermöglicht vorteilhafterweise eine Verbesserung der Messung des Stromsignals durch einen höheren Stromfluss durch das Betriebsmittel 5.

In einer vorteilhaften Ausführungsform ist am Gehäuse 10 eine Anschlagkante 20 angebracht, welche mit einer Innenwand der Vorrichtung zusammenwirkt, um eine Krafteinwirkung beim Erreichen des gesicherten Zustandes auf die Verriegelungsvorrichtung, zu kompensieren.

Ist der gesicherte Verschlusszustand erreicht, wird vorteilhafterweise durch eine Exzenterkante 8 ein Kontaktschalter 1 betätigt. Dadurch wird beispielsweise ein Stromkreis geschlossen und mittels eines optischen und/oder haptischen und/oder akustischen Signals der gesicherte Verschlusszustand angezeigt.

Soll die Tür wieder geöffnet werden, ist der Vorgang im Wesentlichen umzukehren. Durch Aufbringung eines Drehmoments am Exzenter 4 entgegen der Verschlussrichtung V wird die Sperrklinke 2 in den ungesicherten Verschlusszustand gebracht. Dieser ungesicherte Verschlusszustand kann vorteilhafterweise dann durch ein weiteres Drehmoment vom Exzenter 4, insbesondere durch ein Betriebsmittel 5, entgegen der Verschlussrichtung V schließlich in den geöffneten Zustand überführt werden.

Die Fig. 4 und 5 zeigen eine schematische Innenansicht der Verriegelungsvorrichtung zur Verdeutlichung eines Notentriegelungssystems, das weitere Vorteile bringt. Weiterhin wird in Fig. 5 und 6 die Lagerung 17 des Übertragungsmittels 15 verdeutlicht. Die in Fig. 6 dargestellt schematische Seitenansicht verdeutlicht das Funktionsprinzip und die Lagerung 17 des Übertragungsmittels 15 zusätzlich.

Die Lagerung 17 des Übertragungsmittels 15, insbesondere der Welle 16, vorteilhafterweise eine Lagerung durch Gummihüllen, ermöglicht eine Federung und Dämpfung. Dadurch wird beispielsweise einer Beschädigung des Betriebsmittels 5 und des Übertragungsmittels 15, insbesondere der Welle 16, entgegengewirkt, da das Anfahrtsmoment des Betriebsmittels 5 und die Rückwirkungen der Krafteinwirkungen, durch Zusammenwirken der Sicherungskanten 6 und 7 des Exzenters 4 mit den entsprechenden Anschlägen 19 und 23, beim Öffnen und Schließen kompensiert werden. Weiterhin wird mit Vorteil eine Geräuschdämpfung erreicht.

Da der gesicherte Verschlusszustand (siehe Fig. 3) nur durch eine Umkehrung des Drehmoments am Exzenter 4 entgegen der Verschlussrichtung V aufgehoben werden kann, ist die Tür bestimmungsgemäß von außen nicht öffenbar.

Für Notfälle ist es vorteilhaft, ein Notentriegelungssystem bereitzustellen, mit dem von außen über einen anderen Mechanismus ein Drehmoment aufbringbar ist, mit dem der Exzenter 4 oder die Schubstange 2 aus dem gesicherten Verschlusszustand gebracht werden kann.

Der gesicherte Verschlusszustand kann durch ein Drehmoment, beispielsweise eine Notentriegelungskraft N, von außen durch einen Notentriegelungsauslöser 11, insbesondere einen Zuganker (siehe auch Draht für Zuganker 12), aufgehoben werden. Die Verriegelungsvorrichtung wird dadurch in den ungesicherten Verschlusszustand überführt; die Tür kann durch Ziehen geöffnet werden.

Dabei ist es vorteilhaft, dass das Betriebsmittel 5 über keine Selbsthemmung verfügt. Dadurch wird der Notentriegelung nicht entgegengewirkt.

Eine von außen wirkende Kraft wird auf die Schubstange 3 vorteilhafterweise mittels Hebelkraft über einen Notentriegelungshebel 9 übertragen. Die Kraftübertragung kann beispielsweise aber auch mittels Zugkraft über eine, in der Fig. 5 nicht dargestellte, Zugvorrichtung mit entsprechenden Seilwinden erfolgen.

Die Krafteinwirkung von außen auf die Schubstange 3 erfolgt vorteilhafterweise über den Zuganker 12. Diese Anordnung ist nicht zwingend notwendig und kann auch beispielsweise über eine Kurbel oder ein Seil erfolgen.

Die Übertragung eines Drehmoments auf die Schubstange 3 wird mit Vorteil mittels des Notentriegelungshebels 9 in einer Zwangsführung, vorteilhafterweise in einem Zwangsführungskäfig 13 und an einer Zwangsführungskante 21 an der Schubstange 3, erreicht. Durch die Zwangsführung wird eine optimale Kraftübertragung an einem vorteilhaften Angriffspunkt sichergestellt. Durch eine Krafteinwirkung auf den Zuganker 12 wird diese über den Notentriegelungshebel 9 auf die Schubstange 2 übertragen. Das System wird vom gesicherten in den ungesicherten Verschlusszustand überführt.

Im ungesicherten Verschlusszustand ist es möglich ein Drehmoment entgegen der Verschlussrichtung V durch eine Krafteinwirkung auf die Sperrklinke 2 über die Schubstange 3 auf den Exzenter 4 zu übertragen; die Tür könnte in diesem Zustand geöffnet werden. Dadurch ist es z.B. möglich, sowohl durch eine Umkehrung des Drehmoments am Exzenter 4, als auch durch eine Krafteinwirkung auf die Sperrklinke 2, z.B. durch Ziehen an der Tür, das System von dem ungesicherten Verschlusszustand in den geöffneten Verschlusszustand zu überführen.

Der Ausgangszustand des Notentriegelungssystems wird mit Vorteil durch einen Rückführungsmechanismus am Notentriegelungshebel 9, vorteilhafterweise über eine Feder 14, wieder hergestellt.

Die Kraftübertragung mittels des Notentriegelungsauslösers 11 kann vorteilhafterweise sowohl von Hand, aber auch mittels eines Notentriegelungsbetriebsmittels 22, beispielsweise eines Elektromotors, erfolgen. Das Notentriegelungsbetriebsmittels 22 sowie die Notentriegelungsbauteile 11 sind in den Figuren nicht dargestellt. Alternativ sind aber auch magnetische, hydraulische oder pneumatische Antriebe als Notentriegelungsbetriebsmittel 22 verwendbar.

In den Ausführungsformen der Fig. 4 und 5 ist erkennbar, dass die Schubstange 3 als flacher Körper (hier ein Blech) ausgebildet ist, das an dem ebenfalls als flachen Körper des Exzenters 4 (hier ebenfalls aus Blech) flächig anliegt. Somit weisen Exzenter 4 und Schubstange 3 Kontaktflächen auf. Durch dieses flächenmäßige aufeinanderliegen der Kontaktflächen wird eine gegenseitige Führung bewirkt, die die Stabilität der Vorrichtung erhöht. Somit kann es keine gegenseitige Verkantung geben, wenn beide Bauteile verformt werden; beide Bauteile machen unter Belastung die gleiche Verformung durch, dass eine Blockade der Schubstange 3 am Exzenter 4 unabhängig von der Einbaulage der Türverriegelung und Fertigungstoleranzen vermieden wird.

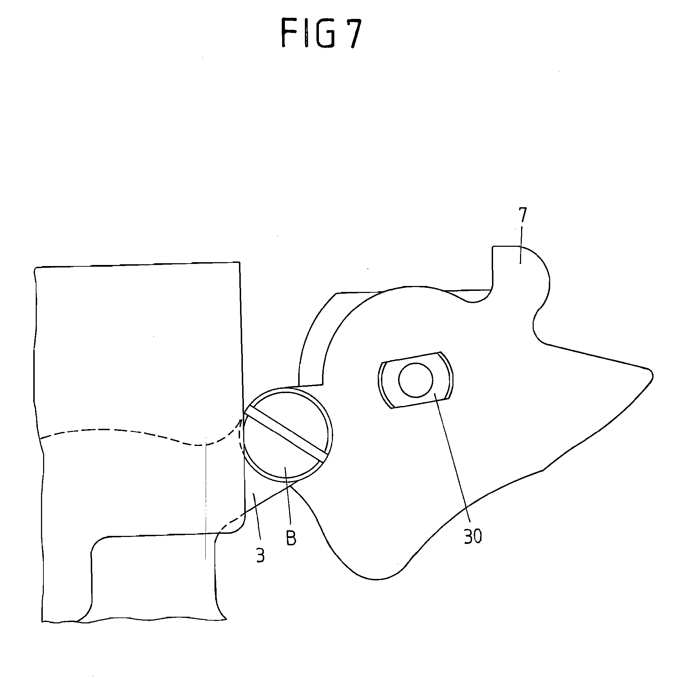

In Fig. 7 ist ein Detail einer Welle-Nabe Verbindung zwischen dem Exzenter 4 und der Antriebswelle 16 dargestellt. Die Welle-Nabeverbindung wird durch ein beidseitig angefrästes Wellenende gebildet, das in eine entsprechend geformte Ausnehmung um Exzenter 4 formschlüssig eingreift. Die Befestigungsschraube und die Unterlegscheibe, die z.B. in Fig. 4 sichtbar ist, ist aus Gründen der Übersichtlichkeit hier weggelassen. In anderen Ausführungsformen kann die Verbindung auch durch anderen Formschluss hergestellt werde, z.B. über eine Vierkantverbindung, eine Sechskantverbindung oder eine nur einseitig abgeflachte Welle. Allen diesen formschlüssigen Verbindungen ist gemeinsam, dass relativ große Drehmomente sicher übertragen werden können. Der Motor 5 bringt ein erhebliches Drehmoment auf, so dass eine stabile Welle-Nabe Verbindung sinnvoll ist.

### Bezugszeichenliste

- 1: Kontaktschalter
- 2: Sperrklinke
- 3: Schubstange
- 4: Exzenter
- 5: Betriebsmittel
- 6: Sicherungskante Exzenter Zustand "offen"
- 7: Sicherungskante Exzenter Zustand "geschlossen"
- 8: Exzenterkante
- 9: Notentriegelungshebel
- 10: Gehäuse
- 11: Notentriegelungsauslöser
- 12: Verbindungsdraht für Zuganker
- 13: Zwangsführungskäfig
- 14: Feder
- 15: Übertragungsmittel
- 16: Welle
- 17: Lagerung
- 18: Verschlussgegenstück
- 19: Anschlag Exzenter Zustand "offen"
- 20: Anschlagkante
- 21: Zwangsführungskante
- 22: Notentriegelungsbetriebsmittel
- 23: Anschlag Exzenter Zustand "geschossen"

- N: Notentriegelungskraft
- V: Verschlussrichtung
- A,B: Drehgelenk

- W1: Verbindungslinie zwischen Exzenter und Sperrklinkengelenk
- W2: Verbindungslinie zwischen Schubstangengelenk und Exzenter

## Patentansprüche

1. Verriegelungsvorrichtung für eine Tür einer Reinigungsvorrichtung, Desinfektionsvorrichtung, Sterilisationsvorrichtung oder Reinigungs- und Desinfektionsvorrichtung
**gekennzeichnet durch**
mindestens eine Sperrklinke (2), mindestens eine Schubstange (3) und mindestens einen Exzenter (4), die **durch** Drehgelenke (A, B) miteinander verbunden sind, wobei bei geschlossener Tür die Drehgelenke (A, B) so angeordnet sind, dass bei einer von außen auf die Tür aufgebrachten Zugkraft ein Drehmoment auf den Exzenter (4) aufgebracht wird, das in der Verschlussrichtung (V) wirkt, so dass ein gesicherter Verschlusszustand vorliegt.

2. Verriegelungsvorrichtung nach Anspruch 1, **dadurch gekennzeichnet, dass** ein Drehmoment auf ein Übertragungsmittel (15), welches zur Übertragung eines Drehmoments auf den Exzenter (4) ausgebildet ist, durch ein Betriebsmittel (5), insbesondere einen Elektromotor, übertragbar ist.

3. Verriegelungsvorrichtung nach Anspruch 2, **dadurch gekennzeichnet, dass** das Übertragungsmittel (15) eine federnde und/oder dämpfende Lagerung (17) aufweist.

4. Verriegelungsvorrichtung nach mindestens einem der vorhergehenden Ansprüche, **dadurch gekennzeichnet, dass** das Drehmoment zum Schließen der Sperrklinke (2) vom Exzenter (4) über die Schubstange (3) auf die Sperrklinke (2) bis zu einem bestimmten Drehwinkel, insbesondere 90°, übertragbar ist und die Verriegelungsvorrichtung in einem ungesicherten Verschlusszustand vorliegt.

5. Verriegelungsvorrichtung nach mindestens einem der vorhergehenden Ansprüche, **dadurch gekennzeichnet, dass** im ungesicherten Verschlusszustand durch Krafteinwirkung auf die Sperrklinke (2) ein Drehmoment auf den Exzenter (4) über die Schubstange (3) entgegen der Verschlussrichtung (V) zum Öffnen der Tür übertragbar ist.

6. Verriegelungsvorrichtung nach mindestens einem der vorhergehenden Ansprüche, **dadurch gekennzeichnet, dass** nach Erreichen des ungesicherten Verschlusszustand das Drehmoment auf den Exzenter (4) und die Schubstange (3) weiter wirken muss, um beide in Verschlussrichtung (V) zu drehen und somit der gesicherte Verschlusszustand erreichbar ist.

7. Verriegelungsvorrichtung nach mindestens einem der vorhergehenden Ansprüche, **dadurch gekennzeichnet, dass** im gesicherten Verschlusszustand durch Krafteinwirkung auf die Sperrklinke (2) kein Drehmoment auf den Exzenter (4) über die Schubstange (3) entgegen der Verschlussrichtung (V) zum Öffnen des Systems übertragbar ist, sondern die Kraft nur in Verschlussrichtung (V) übertragbar ist.

8. Verriegelungsvorrichtung nach mindestens einem der vorhergehenden Ansprüche, **dadurch gekennzeichnet, dass** durch Zusammenwirken einer entsprechend geformten Sicherungskante (6) am Exzenter (4) für den geöffneten Verschlusszustand und/oder einer entsprechend geformten Sicherungskante (7) am Exzenter (4) für den gesicherten Verschlusszustand mit den entsprechenden Anschlägen (19, 23) am Gehäuse (10), eine weitere Krafteinwirkung über den Exzenter (4) kompensierbar ist.

9. Verriegelungsvorrichtung nach mindestens einem der vorhergehenden Ansprüche, **dadurch gekennzeichnet, dass** das Betriebsmittel (5) mittels Messung der Stromstärke und/oder Spannung beim Erreichen einer Obergrenze der Stromstärke und/oder Spannung, insbesondere automatisch, ausschaltbar ist.

10. Verriegelungsvorrichtung nach mindestens einem der vorhergehenden Ansprüche, **dadurch gekennzeichnet, dass** durch Zusammenwirken einer Anschlagkante (20), welche mit einer Innenwand der Vorrichtung zusammenwirkt, eine Krafteinwirkung auf die Verriegelungsvorrichtung, kompensierbar ist.

11. Verriegelungsvorrichtung nach mindestens einem der vorhergehenden Ansprüche, **dadurch gekennzeichnet, dass** im gesicherten Verschlusszustand durch eine Exzenterkante (8) ein Kontaktschalters (1) betätigbar ist und dadurch mittels eines optischen und/oder haptischen und/oder akustischen Signals der gesicherte Verschlusszustand anzeigbar ist.

12. Verriegelungsvorrichtung nach mindestens einem der vorhergehenden Ansprüche, **dadurch gekennzeichnet, dass** der Ausgangszustand eines Notentriegelungssystems zum Auflösen des gesicherten Verschlusszustandes durch einen Rückführungsmechanismus an einem Notentriegelungshebel (9), insbesondere durch eine Feder (14), widerherstellbar ist.

13. Verriegelungsvorrichtung nach mindestens einem der vorhergehenden Ansprüche, **dadurch gekennzeichnet, dass** die Kraftübertragung auf einen Notentriegelungsauslöser (11) eines Notentriegelungssystems zum Auflösen des gesicherten Verschlusszustandes über ein Notentriegelungsbetriebsmittel (22), insbesondere einen Elektromotor, durchführbar ist.

14. Verriegelungsvorrichtung nach mindestens einem der vorhergehenden Ansprüche, **dadurch gekennzeichnet, dass** die Verbindung zwischen Antriebswelle (16) und Exzenter (4) durch Formschluss, insbesondere ein abgeflachtes Wellenende, einen Vierkant oder einen Sechskant erfolgt.

15. Verriegelungsvorrichtung nach mindestens einem der vorhergehenden Ansprüche, **dadurch gekennzeichnet, dass** die Schubstange (3) und der Exzenter (4) im Zusammenbau mindestens teilweise Kontaktflächen zur gegenseitigen Abstützung aufweisen.
